# EUROPÄISCHE PATENTSCHRIFT

(11) **EP 1 342 412 B1**
(45) Veröffentlichungstag und Bekanntmachung des Hinweises auf die Patenterteilung: **26.09.2007**
(21) Anmeldenummer: 03001599.4
(22) Anmeldetag: 24.01.2003
(51) Int. Cl.: A01N 43/56, C07D 231/12

(54) **Rein triklines Metazachlor und Verfahren zu seiner Herstellung**
Triclinic metazachlor and process for its preparation
Métazachlore triclinique et procédé pour sa préparation

(30) Priorität: 09.03.2002 DE 10210409
(43) Veröffentlichungstag der Anmeldung: 10.09.2003
(73) Patentinhaber: Feinchemie Schwebda GmbH, 37269 Eschwege (DE)
(72) Erfinder: Schnell, Michael, Dr., 37269 Eschwege (DE)
(74) Vertreter: Gesthuysen, von Rohr & Eggert

(56) Entgegenhaltungen:
- EP-A- 0 012 216
- EP-A- 0 411 408
- WO-A-96/10913
- WO-A-02/058471

## Beschreibung

Die Erfindung betrifft rein triklines 2-Chlor-(2',6'-dimethyl-N-pyrazol-1-yl-methyl)-acetanilid (Metazachlor) der Formel I, ein Verfahren zur Herstellung dieser rein triklinen Modifikation und diese enthaltende stabile wäßrige Suspensionskonzentrate für die Verwendung als Herbizid.

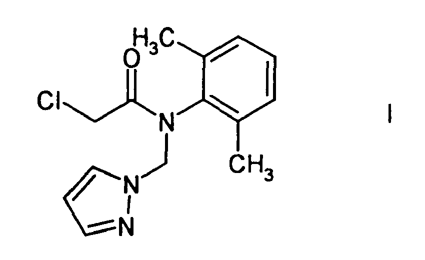

In der WO 96/10913 A1 und der WO 02/058471 A1 sind flüssige Mischungen beschrieben, die Wasser und Metazachlor enthalten. Dort finden sich aber keinerlei Hinweise in bezug auf verschiedene Kristallmodifikationen des Metazachlors und deren Reinheiten.

Der bekannte herbizide Wirkstoff Metazachlor kristallisiert in verschiedenen Kristallmodifikationen, die durch unterschiedliche Schmelzbereiche charakterisiert sind.

In der EP-B-411 408 sind drei Modifikationen von Metazachlor beschrieben:
1. Die trikline Kristallform mit einem Schmelzbereich von 78 bis 83 °C, die nach den in der DE-A-2 648 008, DE-A-2 830 764 und EP-A-12 216 beschriebenen Methoden durch Kristallisation aus einem unpolaren oder wenig polaren Lösungsmittel, wie Cyclohexan oder Toluol, erhältlich sein soll. In Form konzentrierter wäßriger Suspensionen soll diese Modifikation den Nachteil haben, daß sie häufig Agglomerate bildet, so daß ein gleichmäßiges Austragen der formulierten Ware nicht mehr gewährleistet ist.
2. Dieses unerwünschte Verhalten zeigt eine bei 76 °C schmelzende monokline Modifikation nicht. Sie wird erhalten, indem man Metazachlor aus einem polaren organischen Lösungsmittel umkristallisiert und nach vollständiger Kristallisation den Festkörper in üblicher Weise isoliert oder in dem man das Metazachlor aus wäßrig schwefelsaurer Lösung bei Temperaturen von 0 bis 50 °C in Gegenwart eines polaren mit Wasser mischbaren inerten organischen Lösungsmittels ausfällt. Zu beiden Modifikationen werden charakteristische physikalische Daten angegeben.
3. Eine metastabile bei 84 °C schmelzende Metazachlormodifikation, die durch Erwärmen von triklinem Metazachlor auf 84 °C und Anreiben der gebildeten Schmelze erhalten wird. Beim Stehen soll sich diese Kristallform mit einer Halbwertzeit von 5 Tagen in die bei 76 °C schmelzende monokline Modifikation umwandeln.

In den für die Herstellung von triklinem Metazachlor benannten Druckschriften findet sich keine Angabe einer Kristallmodifikation oder einer verlässlichen Methode, aus der sich Aussagen zu einer Kristallmodifikationen ableiten ließen. Die publizierten Schmelzpunkte von 78 bis 83 °C der verschiedenen Synthesebeispiele lassen keinen eindeutigen Schluss zu, welche Metazachlormodifikation erhalten wurde und ob es sich dabei um reine Kristallformen oder um Gemische verschiedener Modifikationen handelte.
Dabei muss man anmerken, daß eine verlässliche Ermittlung der Kristallform aus dem Schmelzverhalten nur mit Hilfe der Thermoanalyse (DTA oder DSC) möglich ist. Bestimmung des Schmelzpunktes mit einem Schmelzpunktsröhrchen in einem Heizbad oder mit einem Schmelzblock nach Kofler ergeben nur Aussagen zum Anfang und Ende des Schmelzvorganges. Insbesondere erhält man keine Aussagen zu Phasenumwandlungen, zur Schmelzwärme und im Falle von Gemischen auch keine Angabe der prozentualen Zusammensetzung. In den zitierten Patenten wird die Verwendung von DTA oder DSC jedoch nicht erwähnt.

Bei der Überprüfung der Kristallisation von Metazachlor in Toluol, nach der üblichen Methode (Auflösen von Metazachlor in der Wärme und Kristallisation bis zur Raumtemperatur) hat sich nun überraschend herausgestellt, daß entgegen der Aussage der EP 411 408 nach den Verfahren der zitierten Patentanmeldungen kein rein triklines Metazachlor erhalten wird. Es entstehen vielmehr Gemische verschiedener Metazachlor Modifikationen, die in Abhängigkeit von den Kristallisationsbedingungen, insbesondere der Verweildauer bei Raumtemperatur einen Anteil von 1 bis 99 % an monoklinem Metazachlor aufweisen. Weiter wurde gefunden, daß solche Mischungen von Metazachlormodifikationen, insbesondere beim Vermahlen, beim Rühren in Wasser oder einem organischem Lösungsmittel einer langsamen aber stetig verlaufenden Umlagerung von triklinem zu monoklinem Metazachlor unterliegen. Wird bei diesen Versuchen fein gemahlenes Metazachlor eingesetzt, findet zusätzlich noch eine Zunahme der Korngröße statt, da das neu gebildete, monokline Metazachlor in kristalliner Form entsteht.

Überraschend wurde nun gefunden, daß rein triklines Metazachlor erhalten wird, wenn man eine übersättigte Lösung von Metazachlor in einem organischen Lösungsmittel im Temperaturbereich von 45 bis 75 °C auskristallisieren lässt und nach vollständiger Kristallisation den Festkörper in üblicher Weise isoliert.
Die Temperatur beträgt bei der Kristallisation vorzugsweise 50 bis 70 °C, insbesondere 55 bis 60 °C.

Dabei können sowohl unpolare wie auch polare Lösungsmittel eingesetzt werden. Das Lösungsmittel oder Lösungsgemisch sollte zweckmäßig so gewählt werden, daß es im Temperaturbereich von 50 bis 70 °C nicht mehr als 100 g, vorzugsweise nicht mehr als 80 g, insbesondere nicht mehr als 60 g Metazachlor pro Liter löst. Es werden also Lösungsmittel bevorzugt, die nur ein geringes Lösungsvermögen in dem für die Kristallisation gewünschten Temperaturbereich aufweisen.
Lösungsmittel, in denen sich Metazachlor sehr gut löst, lassen sich dennoch verwenden, wenn durch Zugabe eines zweiten Lösungsmittels mit geringem Löslichkeitsvermögen wie beispielsweise aliphatische Kohlenwasserstoffe das Löslichkeitsvermögen entsprechend verringert wird. Dabei ist es möglich, das zweite Lösungsmittel sofort oder erst während der Kristallisation portionsweise oder kontinuierlich zuzusetzen.

Als unpolare Lösungsmittel eignen sich insbesondere acyclische oder cyclische aliphatische Kohlenwasserstoffe wie Petrolether, Ligroin, Cyclohexan, aromatische Kohlenwasserstoffe wie Toluol, Xylol, Cumol oder aromatenhaltige, technische Lösungsmittelgemische wie Solvesso^{®} oder Shellsol^{®}.

Als polare Lösungsmittel eignen sich insbesondere Alkohole wie Methanol, Ethanol, Isopropanol, n-Propanol, Butanol, Isobutanol, tert.-Butanol, Oktanol, Ethylenglykol, Propylenglykol und Butandiole, Ketone wie Aceton und Butan-2-on, Ether wie Diisopropylether, Tetrahydrofuran und 1,4-Dioxan, Amide wie Dimethylformamid und N-Methylpyrrolidon, sowie Dimethylsulfoxid und Diethylenglykol.

Das erfindungsgemäß hergestellte Metazachlor liegt in einer rein triklinen Modifikation vor. Die Abwesenheit der anderen Kristallformen wurde durch Thermoanalyse (Differential Scanning Calorimetry, DSC) Abb. 1, durch ein Röntgenpulverdiffraktogramm Tabelle 1 und durch einen Stabilitätstest Tabelle 2 festgestellt. Abwesenheit der anderen Kristallformen bedeutet, daß die monokline und die metastabile, bei 84 °C schmelzende Kristallform durch die genannten Analysenverfahren nicht mehr nachweisbar sind und höchstens 0,5 %, insbesondere nicht mehr als 0,2 % ausmachen.

Das erfindungsgemäße rein trikline Metazachlor zeichnet sich durch folgende charakteristische physikalische Daten aus:

| Meßmethode | Parameter | Ergebnis | Dimension |
|---|---|---|---|
| Thermoanalyse (DSC) | Onset Schmelzwärme | 80,2-80,8 84-86 | °C J/g |
| Röntgenpulverdiffraktogramm | Beugungswinkel | Tabelle 1 | 2 ⊖ |
| Mikroskop | Kristallform | Prismatisch | |

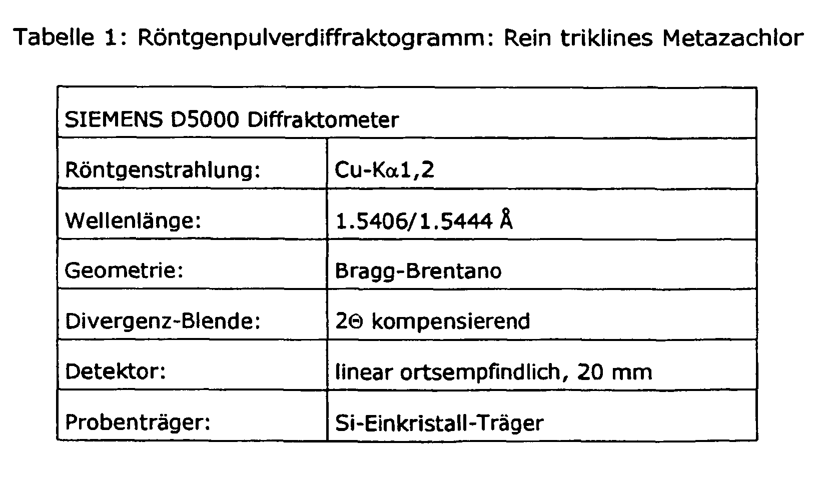

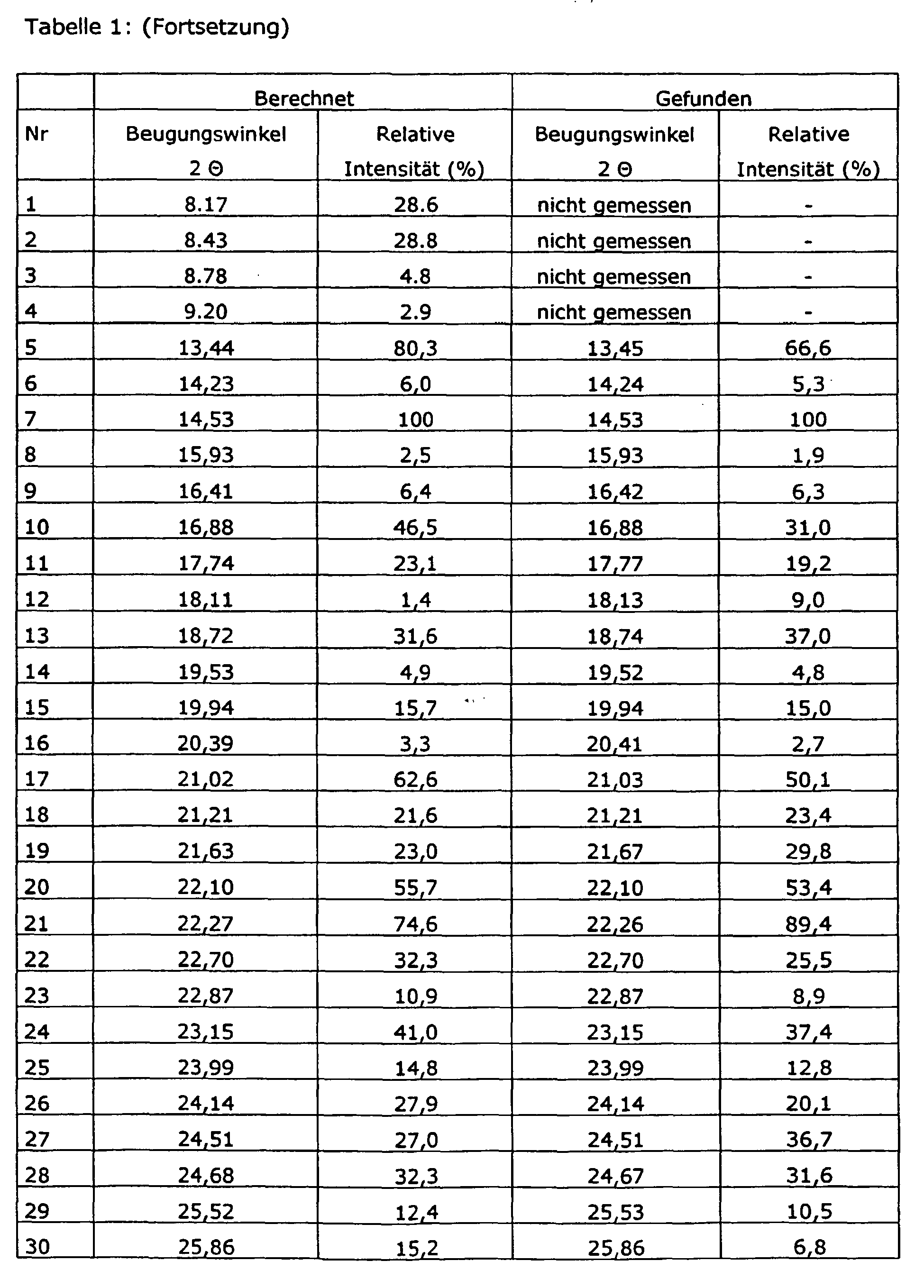

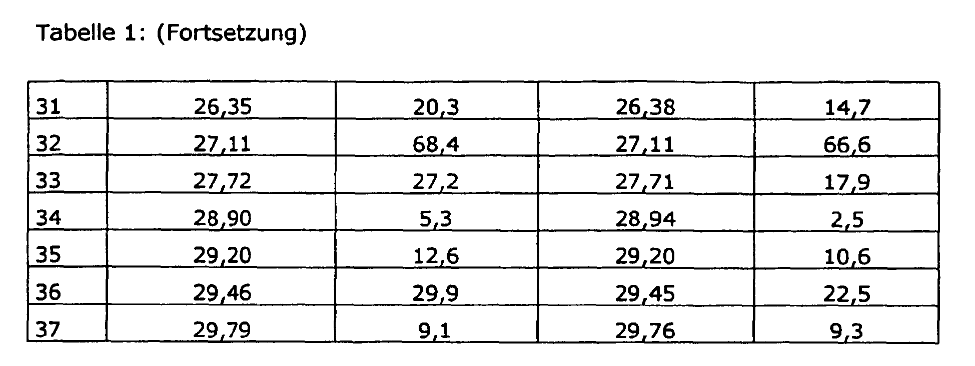

### Tabelle 2:

Stabilitätstest für rein triklines Metazachlor, Beispiele 1-4. 5,0 g Metazachlor werden 24 Stunden bei 25°C in 15 g Wasser mit einem Magnetrührwerk gerührt. Man saugt das Metazachlor ab und trocknet an der Luft. Die Modifikation wird durch Thermoanalyse (DSC) anhand des Schmelzverhaltens ermittelt. Im Falle von Gemischen wird die Zusammensetzung aus dem Verhältnis der Schmelzflächen bestimmt. Eine rein trikline Modifikation erkennt man daran, daß nach 24 Stunden keine monokline Modifikation nachgewiesen werden kann (Beispiel 1). Schon geringe Zusätze von monoklinem Metazachlor bewirken, daß sich die gesamte Kristallmasse mehr oder weniger vollständig in die monokline Modifikation umwandelt, was sich leicht am Schmelzbereich erkennen lässt (Beispiele 2-4).

| | Vor dem Stabilitätstest | | Nach dem Stabilitätstest | | |
|---|---|---|---|---|---|
| | Metazachlor | | DSC 822^{e} | Modifikation in % | |
| Bsp. | Triklin | Monoklin | Onset °C | Triklin | Monoklin |
| 1) | 100 % | - | 80,60 | 100 % | - |
| 2) | 99,5 % | 0,5 % | 75,6 + 80,4 | 99,8 % | 0,2 % |
| 3) | 99 % | 1 % | 74,59 | - | 100 % |
| 4) | 95 % | 5 % | 75,00 | - | 100 % |

Durch Thermoanalyse (DSC) wurden die Schmelzwärmen der beiden Kristallmodifikationen ermittelt und zwar für monoklines Metazachlor 98 - 102 J/g und für triklines Metazachlor 84 - 86 J/g. Monoklines Metazachlor ist demnach die energieärmste Modifikation, die man als energetischen Grundzustand bezeichnen kann. Alle anderen Modifikationen, auch die trikline Modifikation, sind nur metastabile Phasen, die bei Anwesenheit von Impfkristallen durch relativ geringe Anregungsenergien in monoklines Metazachlor umgelagert werden. Das erfindungsgemäße rein trikline Metazachlor ist also vermutlich eine eingefrorene Modifikation ohne Impfkristalle einer anderen Modifikation. Es hat sich überraschenderweise gezeigt, daß Metazachlor in dieser Modifikation vollständig stabil ist. Weder beim Lagern, Trocknen oder nassen Vermahlen noch beim Rühren in Wasser wird eine Umlagerung in die monokline Modifikation beobachtet.

Die Thermoanalyse (DSC) lässt sich mit verschiedenen Aufheizraten durchführen. Wählt man für das monokline Metazachlor eine Aufheizrate von 10 K/min, so findet man einen singulären Schmelzbereich von 76,9 °C (Onset) bis 79,4 °C (Peak), der für monoklines Metazachlor charakteristisch ist (Abb. 2). Wählt man jedoch eine Aufheizrate von 1 K/min so beobachtet man drei getrennte Schmelzbereiche, die dem monoklinen (76,3-77,1 °C), triklinen (80,6-81,0 °C) und "metastabilen" (84,0-84,8 °C) Metazachlor entsprechen (Abb. 3, obere Kurve). Setzt man dem monoklinen Metazachlor 10 % triklines Metazachlor zu und wiederholt das Schmelzexperiment mit der gleichen Aufheizrate von 1 K/min beobachtet man nur noch zwei Schmelzbereiche für wenig monoklines und hauptsächlich triklines Metazachlor (Abb. 3, untere Kurve). Diese unterschiedlichen Resultate sind auf eine Umwandlung des monoklinen Metazachlors in die anderen Modifikation während des Schmelzvorganges zurückzuführen.
Bei der schnellen Aufheizrate von 10 K/min findet das System keine Zeit, die anderen Modifikationen zu bilden. Bei der langsamen Aufheizrate von 1K/min setzt jedoch gleichzeitig mit dem Schmelzen des monoklinen Metazachlors eine Phasenumwandlung zu den höher schmelzenden Modifikationen ein. Deutlich erkennbar ist die Phasenumwandlung durch einen exothermen Bereich zwischen 78-80 °C. Aus diesen Experimenten lässt sich ableiten, daß bei hohen Temperaturen die höher schmelzenden Modifikationen bevorzugt gebildet werden. Sind bereits Impfkristalle der triklinen Modifikation vorhanden, wird dabei ausschließlich die trikline Modifikation gebildet (Abb. 3, untere Kurve).

Aus dem erfindungsgemäßen, rein triklinen Metazachlor lässt sich durch Mischen mit Wasser, oberflächenaktiven Substanzen und weiteren Zusatzstoffen und anschließendem Vermahlen in einer Kugelmühle ein herbizides, wässriges Suspensionskonzentrat mit einem Gehalt von 10 bis 60 Gew.-% an Metazachlor herstellen.

Zur Beurteilung der Lagerstabilität wurde das Suspensionskonzentrat zwei Wochen bei 54 °C (beschleunigter Lager-Test) oder 6 Monate bei 20 °C gelagert. Vor und nach Lagerung wurden der Wirkstoffgehalt, die Suspendierbarkeit, die Kornverteilung, der Siebrückstand und die Modifikation des Metazachlor bestimmt. Bei diesen Untersuchungen wurden keine signifikanten Veränderungen festgestellt (Tabelle 3).

**Tabelle 3: Lagerstabilität eines wässrigen Suspensionskonzentrates (SC), das aus rein triklinem Metazachlor hergestellt wurde.**

| | Vor Lagerung | Nach 2 Wochen bei 54 °C | Nach 6 Monaten bei 20 °C |
|---|---|---|---|
| | | | |
| Aussehen | beigefarbene Flüssigkeit | beigefarbene Flüssigkeit | beigefarbene Flüssigkeit |
| Metazachlor | | | |
| Gehalt | 502 g/l | 502 g/l | 502 g/l |
| Modifikation | Triklin | Triklin | Triklin |
| DSC822^{e} | Onset: 80,6 °C (85,6 J/g) | Onset: 80,0 °C (84,7 J/g) | Onset: 80,0 °C (84,4 J/g) |
| | | | |
| Suspendierbarkeit | In Schwebe befindliches Metazachlor in % zur Gesamtmenge | | |
| Spontan | | | |
| 5 I SC in 100 I Wasser | 94,1 % | 95,1% | 94,5% |
| | | | |
| nach 30 Min. Standzeit | | | |
| 2,5 I SC in 100 I Wasser | 98,7% | 94,2% | 97,0% |
| 1,0 I SC in 220 I Wasser | 99,5% | 97,5% | 98,5% |
| | | | |
| Nasssiebung | Siebrückstand in Gew.% zum Gesamtgewicht | | |
| 75 µm Sieb | 0,03% | 0,07% | 0,05% |
| | | Statistische Verteilung der Partikelgröße | |
| Kornverteilung | 50 % < 1,6 µm | 50 % < 2,8 µm | 50 % < 1,9 µm |
| (Cilas 715 Granulometer) | 90 % < 2,8 µm | 90 % < 3,8 µm | 90 % < 3,0 µm |

Die folgenden Beispiele 1 bis 9 verdeutlichen das erfindungsgemäße Verfahren. 10 g Metazachlor werden in einem Lösungsmittel oder einem Gemisch von Lösungsmitteln bei 60-80 °C gelöst und auf eine Starttemperatur abgekühlt. Zur Auslösung einer gleichmäßigen Kristallisation setzt man ca. 20 mg triklines Metazachlor zu und lässt unter ständigem Rühren bis zur Endtemperatur abkühlen. Die Kristalle werden abgesaugt, getrocknet, gewogen und die Modifikation durch Thermoanalyse (Mettler-Toledo, DSC822^{e}) ermittelt und im Beispiel 6 zusätzlich durch Röntgenpulverdiffraktometrie bestätigt.

Die Onset Temperatur (°C) charakterisiert den Beginn des Schmelzens einer Phase und ist im Gegensatz zur Peak Temperatur weitgehend unabhängig von der Aufheizrate und deshalb besonders geeignet zur Identifikation der Modifikationen. Die Angabe J/g steht für die Schmelzwärme pro Gramm und ist bei einer einheitlichen Phase ebenfalls charakteristisch für die Modifikation.
Triklines Metazachlor Onset = 80 - 81 °C; J/g = 84 - 86 J/g
Monoklines Metazachlor Onset = 75 - 76 °C; J/g = 98 - 102 J/g

**Tabelle 4: Beispiele 1 - 9**

| Nr | Lösungsmittel | ml | Temperatur Start Ende | | Zeit | DSC822^{e} Onset | | Modifikation | Ausbeute | Stabilitäts Test |
|---|---|---|---|---|---|---|---|---|---|---|
| | | | °C | °C | h | °C | J/g | | g | |
| 1 | iOctanol | 10 | 60 | 45 | 24 | 80,56 | 86,4 | triklin | 8,3 | stabil |
| 2 | 2-Butanol iOctan | 5 20 | 60 | 45 | 24 | 80,44 | 86,1 | triklin | 8,0 | stabil |
| 3 | Toluol iOctan | 4 10 | 65 | 45 | 12 | 80,22 | 85,6 | triklin | 8,9 | stabil |
| 4 | Cumol | 7 | 50 | 45 | 12 | 80,70 | 85,2 | triklin | 6,5 | stabil |
| 5 | Cumol iOctan | 7 20 | 60 | 45 | 12 | 80,80 | 86,1 | triklin | 8,9 | stabil |
| 6 | Cyclohexan | 25 | 60 | 45 | 12 | 80,6 | 85,6 | triklin | 9,2 | stabil |
| 7 | Diisopropylether | 10 | 55 | 45 | 12 | 80,30 | 85,6 | triklin | 9,8 | stabil |
| 8 | Solvesso 150 iOctan | 4 10 | 70 | 45 | 24 | 80,34 | 86,1 | triklin | 9,2 | stabil |
| 9 | iPropyl Acetat iOctan | 4 11 | 65 | 45 | 24 | 80,39 | 85,6 | triklin | 9,7 | stabil |

## Patentansprüche

1. Metazachlor (2-Chlor-(2', 6'-dimethyl-N-pyrazol-1-yl-methyl)-acetanilid) der Formel I, **dadurch gekennzeichnet, daß** es nur aus der rein triklinen Modifikation besteht 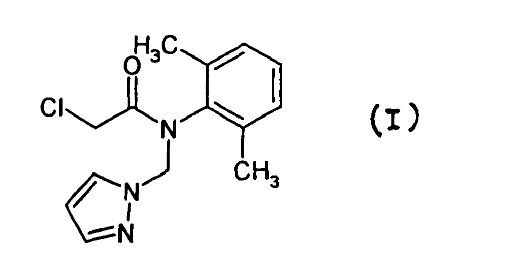 und durch folgende charakteristische physikalische Daten **gekennzeichnet** ist :
| Meßmethode | Parameter | Ergebnis | Dimension |
|---|---|---|---|
| Thermoanalyse (DSC) | Onset Schmelzwärme | 80,2-80,8 84-86 | °C J/g |
| Röntgenpulverdiffraktogramm | Beugungswinkel | Tabelle 1 | 2 Θ |
| Mikroskop | Kristallform | Prismatisch | |

2. Rein triklines Metazachlor gemäß Anspruch 1, **dadurch gekennzeichnet, daß** andere Modifikationen des Metazachlors höchstens 0,5 %, bevorzugt höchstens 0,2 % betragen.

3. Verfahren zur Herstellung der Verbindung nach einem der Ansprüche 1 und 2, **dadurch gekennzeichnet, daß** man eine übersättigte Lösung von Metazachlor aus einem organischen Lösungsmittel im Temperaturbereich von 45 bis 75 °C auskristallisieren lässt und nach vollständiger Kristallisation den Festkörper in üblicher Weise isoliert.

4. Verfahren nach Anspruch 3, **dadurch gekennzeichnet, daß** die Temperatur bei der Kristallisation 50 bis 70 °C, vorzugsweise 55 bis 60 °C, beträgt.

5. Verfahren nach Anspruch 3 oder 4, **dadurch gekennzeichnet, daß** das organische Lösungsmittel oder Lösungsmittelgemisch im Temperaturbereich von 50 bis 70 °C nicht mehr als 100 g Metazachlor pro Liter, vorzugsweise nicht mehr als 80 g Metazachlor pro Liter, löst.

6. Verfahren nach Anspruch 5, **dadurch gekennzeichnet, daß** das Lösungsmittel oder Lösungsmittelgemisch im Temperaturbereich von 50 bis 70 °C nicht mehr als 60 g Metazachlor pro Liter löst.

7. Verfahren nach einem der Ansprüche 3 bis 6, **dadurch gekennzeichnet, daß** als Lösungsmittel unpolare Lösungsmittel aus der Gruppe von acyclischen oder cyclischen aliphatischen Kohlenwasserstoffe wie Petrolether, Ligroin, Cyclohexan, aromatischen Kohlenwasserstoffen wie Toluol, Xylol, Cumol oder aromatenhaltigen, technischen Lösungsmittelgemischen wie Solvesso® oder Shellsol® gewählt werden.

8. Verfahren nach einem der Ansprüche 3 bis 6, **dadurch gekennzeichnet, daß** als Lösungsmittel polare Lösungsmittel aus der Gruppe von Alkoholen wie Methanol, Ethanol, Isopropanol, n-Propanol, Butanol, Isobutanol, tert.-Butanol, Oktanol, Ethylenglykol, Diethylenglykol, Propylenglykol und Butandiolen, Ketonen wie Aceton und Butan-2-on, Ethern wie Diisopropylether, Tetrahydrofuran und 1,4-Dioxan, Amiden wie Dimethylformamid und N-Methylpyrrolidon, sowie Dimethylsulfoxid gewählt werden.

9. Verfahren nach einem der Ansprüche 3 bis 8, **dadurch gekennzeichnet, daß** als Lösungsmittel Gemische der in den Ansprüchen 7 und 8 definierten polaren und unpolaren Lösungsmittel im Verhältnis 1:2 bevorzugt 1:4 und insbesondere 1:6 eingesetzt werden.

10. Verfahren nach einem der Ansprüche 3 bis 9, **dadurch gekennzeichnet, daß** man die Kristallisation im polaren Lösungsmittel oder einer Mischung aus polaren und unpolaren Lösungsmitteln beginnt und unpolares Lösungsmittel während der Kristallisation portionsweise oder kontinuierlich zusetzt.

11. Verfahren nach einem der Ansprüche 3 bis 10, **dadurch gekennzeichnet, daß** man die Kristallisation durch Zugabe von Impfkristallen der Verbindung nach einem der Ansprüche 1 oder 2 auslöst.

12. Stabiles, wäßriges Suspensionskonzentrat, enthaltend 10 bis 60 Gew.-% der Verbindung nach einem der Ansprüche 1 oder 2 und übliche Zusatzstoffe.

## Claims

1. Metazachlor (2-chloro(2',6'-dimethyl-N-pyrazol-1-yl-methyl)acetanilide) of the formula I, **characterized in that** it consists only of the purely triclinic modification 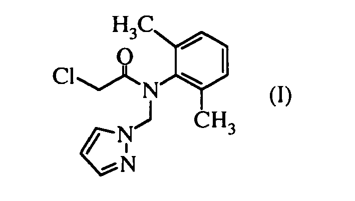 and is **characterized by** the following characteristic physical data:
| Method of measurement | Parameter | Result | Dimension |
|---|---|---|---|
| Thermal analysis (DSC) | Onset | 80.2-80.8 | °C |
| | Heat of fusion | 84-86 | J/g |
| X-ray powder diffraction pattern | Diffraction angle | Table 1 | 2 Θ |
| Microscope | Crystal form | Prismatic | |

2. Purely triclinic metazachlor according to Claim 1, **characterized in that** other modifications of metazachlor account for not more than 0.5 %, preferably not more than 0.2 %.

3. Process for the preparation of the compound according to either of Claims 1 and 2, **characterized in that** a supersaturated solution of metazachlor is allowed to crystallize from an organic solvent in the temperature range of 45 to 75 °C and, after complete crystallization, the solid is isolated in the customary manner.

4. Process according to Claim 3, **characterized in that** the temperature during the crystallization is 50 to 70 °C, preferably 55 to 60 °C.

5. Process according to Claim 3 or 4, **characterized in that** the organic solvent or solvent mixture dissolves not more than 100 g of metazachlor per litre, preferably not more than 80 g of metazachlor per litre, in the temperature range of 50 to 70 °C.

6. Process according to Claim 5, **characterized in that** the solvent or solvent mixture dissolves not more than 60 g of metazachlor per litre in the temperature range of 50 to 70 °C.

7. Process according to any of Claims 3 to 6, **characterized in that** nonpolar solvents from the group consisting of acyclic or cyclic aliphatic hydrocarbons, such as petroleum ether, ligroin or, cyclohexane, aromatic hydrocarbons, such as toluene, xylene or cumene, or aromatics-containing, industrial solvent mixtures, such as Solvesso^{®} or Shellsol^{®}, are chosen as solvents.

8. Process according to any of Claims 3 to 6, **characterized in that** polar solvents from the group consisting of alcohols, such as methanol, ethanol, isopropanol, n-propanol, butanol, isobutanol, tert-butanol, octanol, ethylene glycol, diethylene glycol, propylene glycol and butanediols, ketones, such as acetone and butan-2-one, ethers, such as diisopropyl ether, tetrahydrofuran and 1,4-dioxane, amides, such as dimethylformamide and N-methylpyrrolidone, and dimethyl sulphoxide are chosen as solvents.

9. Process according to any of Claims 3 to 8, **characterized in that** mixtures of the polar and nonpolar solvents defined in Claims 7 and 8 are used in the ratio 1:2, preferably 1:4 and in particular 1:6 as solvents.

10. Process according to any of Claims 3 to 9, **characterized in that** the crystallization is begun in the polar solvent or a mixture of polar and nonpolar solvents, and nonpolar solvent is added in portions or continuously during the crystallization.

11. Process according to any of Claims 3 to 10, **characterized in that** the crystallization is initiated by adding seed crystals of the compound according to either of Claims 1 and 2.

12. Stable, aqueous suspension concentrate containing 10 to 60 % by weight of the compound according to either of Claims 1 and 2 and customary additives.

## Revendications

1. Métazachlore (2-chloro-(2',6'-diméthyl-N-pyrazol-1-ylméthyl)-acétanilide) de formule 1, **caractérisé en ce qu'**il est constitué uniquement de la modification triclinique pure 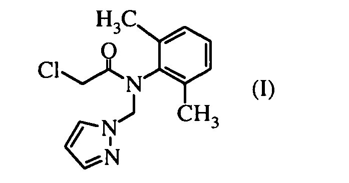 et qu'il est **caractérisé par** les données physiques caractéristiques suivantes :
| Méthode de mesure | Paramètre | Résultat | Dimension |
|---|---|---|---|
| Analyse thermique (ACD) | Début | 80,2-80,8 | °C |
| | Chaleur de fusion | 84-86 | J/g |
| Diffiactogramme de rayons X sur poudre | Angle de diffraction | Tableau 1 | 2 Θ |
| Microscope | Forme cristalline | Prismatique | |

2. Métazachlore triclinique pur selon la revendication 1, **caractérisé en ce que** d'autres modifications du métazachlore représentent au plus 0,5 %, de préférence au plus 0,2 %.

3. Procédé de préparation du composé selon l'une ou l'autre des revendications 1 ou 2, **caractérisé en ce qu'**on laisse une solution sursaturée de métazachlore cristalliser dans un solvant organique dans la plage de températures de 45 à 75 °C et qu'on isole le solide d'une façon habituelle une fois la cristallisation terminée.

4. Procédé selon la revendication 3, **caractérisé en ce que** la température lors de la cristallisation est 50 à 70 °C, de préférence de 55 à 60 °C.

5. Procédé selon la revendication 3 ou 4, **caractérisé en ce que** le solvant ou le mélange de solvants organique ne dissout pas plus de 100 g de métazachlore par litre, de préférence, pas plus de 80 g de métazachlore par litre, dans la plage de températures de 50 à 70 °C.

6. Procédé selon la revendication 5, **caractérisé en ce que** le solvant ou le mélange de solvants ne dissout pas plus de 60 g de métazachlore par litre dans la plage de températures de 50 à 70 °C.

7. Procédé selon l'une quelconque des revendications 3 à 6, **caractérisé en ce que** des solvants non polaires parmi le groupe constitué d'hydrocarbures aliphatiques acycliques ou cycliques, tels que l'éther de pétrole, la ligroïne, le cyclohexane, d'hydrocarbures aromatiques, tels que le toluène, le xylène, le cumène, ou de mélanges de solvants industriels comprenant des composés aromatiques, tels que le Solvesso^{®} ou le Shellsol^{®}, sont choisis en tant que solvants.

8. Procédé selon l'une quelconque des revendications 3 à 6, **caractérisé en ce que** des solvants polaires parmi le groupe constitué d'alcools, tels que le méthanol, l'éthanol, l'isopropanol, le n-propanol, le butanol, l'isobutanol, le tert-butanol, l'octanol, l'éthylèneglycol, le diéthylèneglycol, le propylène-glycol et des butanediols, de cétones, telles que l'acétone et la butan-2-one, d'éthers, tels que l'éther diisopropylique, le tétrahydrofurane et le 1,4-dioxane, d'amides, tels que le diméthylformamide et la N-méthylpyrrolidone, ainsi que du diméthylsulfoxyde, sont choisis en tant que solvants.

9. Procédé selon l'une quelconque des revendications 3 à 8, **caractérisé en ce que** des mélanges des solvants polaires et non-polaires définis dans les revendications 7 et 8 sont utilisés en un rapport de 1:2, de préférence de 1:4 et, en particulier, de 1:6, en tant que solvants.

10. Procédé selon l'une quelconque des revendications 3 à 9, **caractérisé en ce que** la cristallisation est commencée dans le solvant polaire ou un mélange de solvants polaires et non polaires, et un solvant non polaire est ajouté par portions ou en continu durant la cristallisation.

11. Procédé selon l'une quelconque des revendications 3 à 10, **caractérisé en ce que** la cristallisation est amorcée en ajoutant des germes cristallins du composé selon l'une ou l'autre des revendications 1 ou 2.

12. Concentré en suspension aqueuse stable comprenant de 10 à 60 % en poids du composé selon l'une ou l'autre des revendications 1 ou 2, et des additifs habituels.
